**Europäisches Patentamt**

**European Patent Office**

**Office européen des brevets**

(11) Veröffentlichungsnummer: **0 007 979**
**B1**

(12) **EUROPÄISCHE PATENTSCHRIFT**

(45) Veröffentlichungstag der Patentschrift: **11.11.81**

(21) Anmeldenummer: **79101615.7**

(22) Anmeldetag: **28.05.79**

(51) Int. Cl.³: **C 07 C 47/544,**
**C 07 C 45/38, B 01 J 23/50**

(54) Verfahren zur Herstellung von Terephthal-, Isophthal- und Phthaldialdehyd.

(30) Priorität: **03.08.78 DE 2834051**

(43) Veröffentlichungstag der Anmeldung:
**20.02.80 Patentblatt 80/4**

(45) Bekanntmachung des Hinweises auf die Patenterteilung:
**11.11.81 Patentblatt 81/45**

(84) Benannte Vertragsstaaten:
**BE CH DE FR GB IT NL**

(56) Entgegenhaltungen:
**DE - A - 2 339 086**
**DE - A - 2 639 342**
**DE - B - 1 143 801**
**US - A - 3 597 485**
**US - A - 3 843 732**

**Ann. *311* (1900)**
**Seiten 353—362**
**Ber. *54* (1921)**
**Seiten 2888—2890**
**Compt. rend. *170* (1920)**
**Seiten 258—261**
**Compt. rend. *171* (1920)**
**Seite 652**

**HOUBEN-WEYL, Methoden der organischen Chemie, 4. Auflage, Bd. 7/1**
**Seiten 159—166**

**HOUBEN-WEYL, Methoden der organischen Chemie, 4. Auflage, Bd. 4/2**
**Seiten 154—162**

**Journ. für prakt. Chemie *151* (1938)**
**Seite 254**

(73) Patentinhaber: **DYNAMIT NOBEL AKTIENGESELLSCHAFT**
**Patentabteilung Postfach 1209**
**D-5210 Troisdorf, Bez. Köln (DE)**

(72) Erfinder: **Bernhardt, Günther, Dr.**
**Rheinstrasse 33**
**D-5205 St. Augustin 2 (DE)**
Erfinder: **Daum, Gerhard, Dr.**
**Erkerstrasse 15c**
**D-5000 Köln 90 (DE)**

# 0 007 979

## Verfahren zur Herstellung von Terephthal-, Isophthal- und Phthaldialdehyd

Gegenstand der Erfindung ist ein Verfahren zur Herstellung der isomeren Phthaldialdehyde aus den entsprechender Xylylenglykolen.

Es ist bekannt, Terephthal- und Isophthaldialdehyd durch alkalische Hydrolyse von Xylylentetrahalogeniden nach der DE—OS 2 339 086 herzustellen, jedoch sind die Nebenprodukte zahlreich und deren Abtrennung schwierig.

Terephthal- und Isophthaldialdehyd können durch Oxydation von Xylol in flüssiger Phase oder Dampfphase z.B. mit Hilfe von Chromsäure nach J. Thiele und E. Winter, Ann. 311 (1900) 353 oder US—PS 3 597 485 hergestellt werden, jedoch ist die Aldehydausbeute sehr niedrig und große Mengen von Chrom-III—Oxydschlamm sind hinderlich.

Die Herstellung von Terephthal- und Isophthaldialdehyd durch Reduktion von Terephthaloxy- und Isophthaloylchloriden mit Wasserstoff in Gegenwart eines Palladium-Katalysators (K. W. Rosenmund, Ber. *54* (1921) 2888) ist wegen großer Katalysatormengen unwirtschaftlich.

Die Oxydation von Xylylenglykolen mit Selendioxid nach Berichte *64* (1930) 261 ist wegen hoher Kosten des Oxydationsmittels auf das Laboratorium beschränkt.

Die Oxydation mit Salpetersäure nach Journ. für prakt. Chem. *151* (1938) 254 erfordert große Überschüsse an Salpetersäure und ist wegen stark exothermem Verlauf der Umsetzung schwer beherrschbar.

Zahlreiche Aldehyde sind durch Gasphasendehydrierung von Alkoholen mit Katalysatoren aus u.a. Kupfer, Silber oder Zinkverbindungen zugängig (Houben-Weyl, Methoden der organischen Chemie, 4. Auflage, Bd. 7/1, S. 160—166).

Dieses Verfahren ist jedoch im wesentlichen auf niedrigsiedende Alkohole wie Methanol oder Butanol beschränkt, da nur bei diesen gute Ausbeuten der Aldehyde erreichbar sind.

Bei höheren Alkoholen (ebenda S. 164) tritt merkliches Absinken der Aldehydausbeute ein sowie Verteeren des Kontakts. Kohlenmonoxidabspaltung sowie Esterbildung werden schon merklich.

Benzylalkohole (ebenda S. 166) neigen bei hohen Dehydrierungstemperaturen zur Ätherbildung und spalten leichter als aliphatische Alkohole Kohlenmonoxid ab.

Es wurde zwar vorgeschlagen, die Dehydrierung im Vakuum und bei Temperaturen von kleiner 300°C und in Anwesenheit von Sauerstoff durchzuführen, wobei die Sauerstoffmenge weniger als 50% derjenigen betragen muß, die zum Abfangen der durch Dehydrierung freisetzbaren Wasserstoffe erforderlich ist (R. R. Davies und H. H. Hogson, Soc. *1943*, 282—284, C. Mouren und G. Mignonac, Compt. rend. *170* (1920), 258—261, Compt. rend. *171* (1920) 652). Diese Maßnahmen erhöhen zwar die Ausbeuten, bewirken aber auch durch die niedrige Dehydrierungstemperatur und das angelegte Vakuum eine hohe Verdünnung des Reaktionsgemisches sowie niedrige Raum-Zeit-Ausbeuten und ergeben daher unwirtsschaftliche Verfahren.

Nach der US—PS 3 843 732 ist die Herstellung von Aldehyden aus Methylaromaten, Benzylalkohol oder z.B. Styrol mit Li-Sn-Phosphor-Katalysatoren möglich, jedoch ist die Ausbeute an Aldehyden gering und die Bildung mehrerer Nebenprodukte sowie die Totaloxydation zu CO und $CO_2$ hoch.

Die Herstellung von Dialdehyden aus Glykolen durch Dehydrierung ist nicht bekannt. Die hierbei erforderliche Dehydrierung beider Hydroxylgruppen des Moleküls zu Aldehydgruppen läßt schlechte Ausbeuten und eine erhebliche Erhöhung der Anzahl der Nebenprodukte erwarten.

Die Herstellung anderer als der einfach aufgebauten Dialdehyde läßt besondere Schwierigkeiten erwarten.

Der vorliegenden Erfindung liegt die Aufgabe zugrunde, ein wirtschaftliches, auch im technischen Maßstab einfach durchführbares Verfahren zur Verfügung zu stellen, das die Herstellung von Phthaldialdehyden mit hoher Raum-Zeit-Ausbeute ermöglicht.

Die Aufgabe wird erfindungsgemäß dadurch gelöst, daß man Xylylenglykole in der Gasphase in Gegenwart eines Silberkatalysators oder gegebenenfalls Kupferkatalysators unter Normaldruck bei 250 bis 500°C umsetzt, wobei zusätzlich als Hilfsstoffe Wasserdampf und Sauerstoff anwesend sind und die Phthaldialdehyde aus den wäßrigen Kondensaten abtrennt und durch Destillation oder Umkristalisation rein gewinnt.

Demgemäß erfolgt die Herstellung in Gegenwart von Wasserdampf, was ungewöhnlich ist.

Nach dem Verfahren können insbesondere p- und m-Xylylenglykol umgesetzt werden.

Bevorzugt sind Temperaturen von 300 bis 450°C. Es ist bevorzugt, daß die Menge des Sauerstoffs mindestens 50%, aber höchstens 150%, sehr bevorzugt 100 bis 130%, der Menge beträgt, die zum Abfangen des durch Dehydrierung des aus Xylylenglykol freisetzbaren Wasserstoffs erfordlich ist.

Das Molverhältnis Xylylenglykol zu Sauerstoff soll daher 1:0,5 bis 1:1,5, vorzugsweise 1:1 bis 1:1,3 betragen.

Es war nicht vorhersehbar, daß auch bei Verwendung von Sauerstoffmengen, die mehr als 50% derjenigen betragen, die zum Abfangen des durch Dehydrierung freisetzbaren Wasserstoffs erforderlich ist, eine erhebliche Steigerung des Xylylenglykolumsatzes und der Phthalaldehydausbeuten erreicht wird, da andererseits nach Houben Weyl: Methoden der organischen Chemie (1954) Band 7/1, Seite 164 eine Sauerstoffmenge, die mehr als 50% der erforderlichen beträgt, die Aldehydausbeuten auf Kosten einer Weiteroxidation zur Säure drastisch erniedrigt.

Ohne die zusätzliche Verwendung von Wasserdampf tritt auch bei dem vorliegenden Verfahren mit Sauerstoffmengen von mehr als 50% der erforderlichen die Weiteroxydation der Phthalaldehyde zu den Dicarbonsäuren oder Phthalaldehydsäuren und sogar Totaloxydation zu Kohlenmonoxyd oder -dioxyd ein. Wasserdampf allein in Abwesenheit von Sauerstoff bewirkt im wesentlichen nur eine Teildehydrierung von Xylylenglykolen zu Hydroxymethylbenzaldehyden. Die gleichzeitige Anwesenheit von Sauerstoff und Wasserdampf bei der Dehydrierung von Xylylenglykol ist daher eine wesentliche Maßnahme.

Der bei der Dehydrierung eingesetzte Sauerstoff kann in reiner Form oder in Abmischung mit inerten Gasen, vorteilhaft jedoch in Form von Luft verwandt werden.

Der eingesetzte Wasserdampf sollte frei sein von sauren oder alkalischen Bestandteilen. Es kann für die Dehydrierung von Xylylenglykolen von Vorteil sein, wenn ein Teil des Wasserdampfes während der Dehydrierung im Reaktor durch Verdampfen von Wasser erzeugt wird. Das Molverhältnis von Xylylenglykol zu Wasser soll zwischen 1:1 und 1:100, vorzugsweise zwischen 1:5 bis 1:50 betragen.

Als Dehydrierungskatalysator eignen sich besonders Silberkatalysatoren, wie sie in den "Methoden der organischen Chemie" von Houben-Weyl, 4. Auflage, Band 4/2, Seiten 154—162 und Band 7/1, Seite 162 ff., beschrieben sind.

Silber kann in Form von Spänen, Granulat, Wolle, Drahtnetzen oder als Schwammsilber eingesetzt werden. Vorteilhaft wird der Katalysator auf einen Träger wie Bimsstein, Siedesteine oder granulierte Kieselsäure oder Aluminiumoxyd aufgebracht. Das Aufbringen des Silbers auf den Träger kann durch Tränken mit geeigneten Silbersalzlösungen oder Aufbringen von feuchtem Silberoxydhydrat, Trocknen und Reduzieren im Wasserstoff/Stickstoffstrom bei 200°C bis 250°C oder durch Erhitzen im Sauerstoffstrom oberhalb der Zersetzungspunkte der Salze oder des Oxydes durchgeführt werden.

Silberkatalysatoren können bei dem vorliegenden Verfahren beliebig oft durch Überleiten von Luft oder Sauerstoff bei Temperaturen von 300°C bis 500°C leicht und in kurzer Zeit regeneriert werden. Der Reaktor kann mit zwei Katalysatorbetten ausgerüstet werden, die wechselweise zur Dehydrierung benutzt und regeneriert werden. Auch weitere Metallkatalysatoren z.B. Kupferkatalysatoren sind an Stelle von Silber einsetzbar.

Kupferkatalysatoren werden in Anwesenheit von Sauerstoff nach kurzer Zeit infolge Oxydbildung inaktiv und müssen zudem mit Wasserstoff regeneriert werden.

Die Verwendung von Kupferkatalysatoren zur Dehydrierung von Xylylenglykolen ist daher möglich, aber nicht bevorzugt.

Praktisch geht man bei der Dehydrierung von Xylylenglykolen so vor, daß man den Xylylenglykoldampf in Mischung mit Wasserdampf und Luft über den vorgeheizten Katalysator leitet. Auch die getrennte Einleitung von Luft und Wasser bzw. Wasserstoff ist möglich. Der Katalysator ist bevorzugt als Festbett angeordnet, kann aber auch in einem Wirbelbett verwendet werden. Als Reaktor dient vorzugsweise ein vertikal oder geneigt angeordneter Röhrenreaktor, dessen Mischzone von außen geheizt wird. Zweckmäßig ist die eigentliche Reaktionszone mit dem Katalysator als von außen heizbar und mittels eines Kühlfingers kühlbar ausgebildet. Die zu dehydrierenden Xylylenglykole können als Schmelzen oder als Lösungen, insbesonders als wäßrige als wäßrige Lösungen in den Reaktor eingespeist werden. Das gemeinsame Einspreisen von Xylylenglykol und Wasser hat den Vorteil, daß die Verdampfung der relativ hochsiedenden Glykole leichter und schonender erfolgt als die Verdampfung von Xylylenglykolen allein, so daß die Bindung von Kondensationsprodukten aus den Glykolen wie auch den Reaktionsprodukten vermieden wird.

Die Verdampfung des Xylylenglykols kann weiter dadurch erleichtert werden, daß zusätzlich mit dem benötigten Sauerstoff bzw. der Luft überhitzter Wasserdampf in den Reaktor geführt wird.

Die zur Verdampfung des Xylylenglykol/Wassergemischs angewandten Temperaturen liegen zwischen 250 und 500°C, vorzugsweise zwischen 300 und 450°C.

Die Reaktion erfolgt im Bereich des Normaldrucks oder bei geringem Unterdruck. Bevorzugt sind daher Drucke von 0,2 bis 1,3 bar.

Die aus dem Reaktor austretenden und mit Wasserdampf verdünnten gasförmigen Phthalaldehyde werden bevorzugt durch gemeinsame Kondensation von Wasserdampf und Produkt in einfacher Weise kondensiert.

Der aus dem Wasser ausgeschiedene Phthalaldehyd kann nach Kühlung durch Filtration abgetrennt und gegebenenfalls durch Destillation, vorzugsweise im Vakuum, oder Umkristallisation rein gewonnen werden.

Die wäßrigen Filtrate enthalten wenig nicht umgesetzte Xylylenglykole und gegebenenfalls kleine Mengen von Hydroxymethylbenzaldehyden, die bei erneutem Einsatz ebenfalls Dialdehyde bilden. Das Filtrat kann nach Aufkonzentration mit frischem Xylylenglykol wieder der Dehydrierung zugeführt werden.

Es ist überraschend, daß die Methylolgruppen des Xylylenglykols fast ausschließlich zu Aldehydgruppen dehydriert werden.

Es ist weiter überraschend, daß bei den hohen Temperaturen von 250 bis 500°C keine Kondensation der Aldehyde zu Polyestern oder Bildung von Polyäthern eintritt. Die Ausbeuten an

**0 007 979**

Phthaldialdehyden und der Umsatz der Xylylenglykole sind ausgezeichnet und können bei geeigneter Reaktionsführung praktisch quantitativ sein. Von besonderem Vorteil ist die nohe Raum-Zeit-Ausbeute, die Umweltfreundlichkeit durch fast völlige Vermeidung von Abluft und Abwasser sowie die Möglichkeit, das Verfahren kontinuierlich auszuführen.

Der Xylylenglykolumsatz errechnet sich nach der Formel:

$$\frac{\text{umgesetztes Xylylenglykol}}{\text{eingesetztes Xylylenglykol}} \cdot 100,$$

die Ausbeute nach der Formel:

$$\frac{\text{gebildetes Dehydrierungsprodukt}}{\text{umgesetztes Xylylenglykol}} \cdot 100.$$

Terephthal- und Isophthaldialdehyd sind wertvolle Zwischenprodukte für organische Synthesen. Sie finden z.B. Verwendung für die Herstellung von optischen Aufhellern, Lichtschutzstabilisatoren und Farbstoffen.

**Beispiel 1**

Auf ein vertikal angeordnetes Stahlrohr der Länge 110 cm und der lichten Weite von 57 mm ist ein Verschluß mit je einer Zuleitung für Gas und Wasserdampf aufgesetzt. Der Boden des Rohres ist durch ein Drahtnetz abgeschlossen, das in der Mitte einen 2,5 cm breiten, kreisförmigen Ausschnitt trägt, durch den ein 30 cm langer Kühlfinger zentrisch hineinragt, so daß zwischen Rohrinnenwand und Kühlfinger eine ringförmig zylindrische Reaktionszone ausgebildet ist (Zone I). Deren unterer Teil ist mit groben Quarzsteinen ausgefüllt, so daß die daraufliegende Füllung von 86 cm³ Katalysator vom Korndurchmesser 0,75 bis 1,6 mm mit dem oberen Ende des Kühlfingers abschließt. Der Katalysator besteht aus 50 Gew.% Silber auf Bimsstein, hergestellt durch Aufrollen von Silberoxid auf den Träger und anschließende Reduktion.

Das freie Volumen der Katalysatorschicht beträgt 52,4 cm³.

Der darüber liegende Zylinderraum dient zum Mischen und Aufheizen der Reaktionsteilnehmer und Hilfsstoffe (Zonen II und III) und ist mit 1 cm langen und 3 mm dicken Stahlzylindern gefüllt.

Die im vorliegenden Fall gleichlangen Zonen II und III werden von außen geheizt. Die Zone I ist sowohl von außen heizbar wie auch durch den Kühlfinger kühlbar ausgebildet. Die Temperaturmessung aller Zonen erfolgt durch Thermoelemente.

Zwischen den Zonen II und III befindet sich seitlich ein bis ins Rohrinnere reichender Kapillarrohr-Anschluß zum Einpumpen der Xylylenglykollösung. Kapillare und Pumpenkopf sind heizbar.

Durch Zuleitungen am Rohrkopf wird Stickstoff mit einer Strömungsgeschwindigkeit von 111,8 l/h und Wasser in Mengen von stündlich 289,6 cm³ eingepumpt. Die Zonen II und III werden aufgeheizt, bis am unteren Ende beider Zonen Temperaturen von 350 bis 355°C herrschen.

Nach Temperatureinstellung wird über das seitliche Kapillarrohr eine auf 70°C erwärmte, 33,3 Gew.-%ige wäßrige p-Xylylenglykollösung eingepumpt, so daß stündlich 132,5 g Xylylenglykol in den Reaktor gefördert werden. Der Stickstoff wird nunmehr durch einen gleichstarken Luftstrom ersetzt. Durch Nachregelung der Heizung der Zone II wird eine Temperatur von 350 bis 355°C aufrechterhalten.

Die Dehydrierungstemperatur in Zone I wird durch alternierendes Heizen und Kühlen auf 395 bis 400°C gehalten.

Nach einer Laufdauer von 22 Stunden wird die Zudosierung von Xylylenglykollösung gestoppt, die über Kopf zugepumpte Wassermenge auf 555 cm³/h erhöht und die zugeführte Luft unter Erhalt der Strömungsgeschwindigkeit innerhalb einer halben Stunde sukzessive durch Stickstoff ersetzt.

Insgesamt wurden 1,04 Mol $O_2$ je Mol Ausgangsstoff zugesetzt.

Der in der Vorlage erstarrte gelblichbraune Terephthaldialdehyd wird von der wäßrigen Mutterlauge durch Filtration getrennt, mit Wasser gewaschen und getrocknet. Der Schmelzpunkt beträgt 111,5—116°C. Durch Aufkonzentration von Filtrat und Waschflüssigkeit auf 1/3 des Volumens wird weiterer Dialdehyd gewonnen. Die Gesamtauswaage beträgt 2,15 kg, die Reinheit 98,1%. Durch Vakuumdestillation ($Kp_{13}$:128—129°C) wird daraus ein farbloses Produkt mit einer Reinheit von mehr als 99,5% erhalten.

Die wäßrige Mutterlauge enthält nach gaschromatographischer Analyse unter Zuhilfenahme einer Eichkurve: 149 g p-Xylylenglykol, 409 g p-Hydroxymethylbenzaldehyd und 61 g Terephthaldialdehyd.

Der Xylylenglykolumsatz beträgt 95,0%, die Terephthalaldehydausbeute: 80,0%.

In weiteren gleichartig kontinuierlich ausgeführten Ansätzen wird die Mutterlauge des jeweils vorangehenden Ansatzes mit ihrem Gehalt an p-Hydroxymethylbenzaldehyd anstelle von Wasser verwendet.

Bezogen auf den Umsatz von Xylylengelykol beträgt dann die Ausbeute an Dialdehyd 97 bis 98%.

4

**0 007 979**

### Beispiel 2

Die Dehydrierung nach Beispiel 1 wurde wiederholt, wobei jedoch das halbe Volumen des in Beispiel 1 genannten Katalysators verwendet wurde, die Dehydrierungstemperatur von 410 bis 415°C erhöht und die Luftzufuhr auf 130 l/h gesteigert wurde.

Umsatz Xylylenglykol: 95,2%; Ausbeute Terephthaldialdehyd: 82,1%.

In einem weiteren Ansatz wurde die wäßrige Mutterlauge der obigen Aufarbeitung wiederverwendet, wobei durch Zugabe von p-Xylylenglykol die umgesetzte Menge ersetzt wurde. Diese Lösung wurde auf 8,75 l eingedampft und in 22 Stunden in den Reaktor eingepumpt.

Die Gesamtausbeute an Terephthaldialdehyd betrug 13,27 kg = 96,5%, bezogen auf den p-Xylylenglykol-Gesamtumsatz von 99,0%.

Die Reinheit betrug 97,1%.

Nach Destillation bei vermindertem Druck ($Kp_{15} = 131/132°C$) wurde ein farbloses Produkt (Reinheit > 99,5%, Fp. 116/116,5°C) erhalten.

### Beispiel 3

Durch den in Beispiel 1 beschriebenen Dehydrierungsreaktor, dessen Katalysator jedoch aus 130 $cm^3$ eines Silber-Bimsstein-Katalysators mit 30 Gew.-% Silber mit einem Korndurchmesser von 1,2 bis 1,8 mm bestand, wurde innerhalb von 22 h eine 33,3 Gew.-%ige wäßrige m-Xylylenglykollösung mit einer Geschwindigkeit eindosiert, daß ein stündlicher Durchsatz von 133 g m-Xylylenglykol durch das Katalysatorbett erfolgte. Zusätzlich wurde Luft mit einer Strömungsgeschwindigkeit von 115 l/h und Wasser in einer Menge von 290,5 $cm^3$/h in Zone III eindosiert.

Die Dehydrierungstemperatur betrug 375 bis 380°C. Die Aufarbeitung des Reaktionsgemisches erfolgte wie in Beispiel 1.

Der Umsatz betrug 98%, die Ausbeute an Isophthaldialdehyd 2,26 kg = 81,3%. Die Reinheit betrug 98,4%.

Nach Vakuumdestillation betrug die Reinheit > 99,5%. Der Schmelzpunkt lag bei 89°C bis 90°C.

### Beispiel 4

Durch den in Beispiel 1 beschriebenen Dehydrierungsreaktor, der eine Katalysatorfüllung von 61,8 $cm^3$ (209,1 g) Silberkristallen (Hersteller Doduco-Chemie) (freies Volumen der Katalysatorschicht 40 ml) enthält, wurde innerhalb von 8 h eine 20 Gew.-%ige p-Xylylenglykol-Lösung so eindosiert, daß ein stündlicher Durchsatz von 66,3 g p-Xylylenglykol durch die Katalysatorzone erfolgte. Zusätzlich wurde Luft mit einer Strömungsgeschwindigkeit von 115 l/h und Wasser in einer Menge von 290 $cm^3$/h in Zone II eindosiert.

Die Dehydrierungstemperatur betrug 390 bis 395°C. Der p-Xylylenglykol-Umsatz betrug 99,4%, die Terephthalaldehyd-Ausbeute: 420 g = 82,1%.

Nach Vakuumdestillation betrug der Schmelzpunkt 115,5—116°C, die Reinheit > 99,5%.

### Beispiel 5

Beispiel 2 wird wiederholt, wobei gleiche Volumen von Katalysatoren verwendet wurden, die a) 35 Gew.-% Silber auf Asbest und b) 25 Gew.-% Silber auf keramischen Formkörpern enthielten.

### Vergleichsbeispiel A

Entsprechend Beispiel 1 wurde eine Dehydrierung von p-Xylylenglykol durchgeführt mit der Abänderung, daß das Molverhältnis von p-Xylylenglykol zu Luftsauerstoff 1:0,2 war.

Der Xylylenglykol-Umsatz betrug 80,5%, die Terephthalaldehydausbeute 998 g = 43,8%.

Wie erkennbar, bewirkt eine verminderte Menge Sauerstoff eine verringerte Ausbeute.

### Vergleichsbeispiel B (ohne Zusatz von Wasser)

Durch die beiden Zuleitungen am Kopf des in Beispiel 1 beschriebenen Reaktors wurden 671 l/h Stickstoff und 120 l/h Luft eingeleitet. In der Katalysatorzone I befanden sich 43 $cm^3$ Silber-Bimsstein-katalysator (Silbergehalt 50 Gew.-%) der Körnung 0,75 bis 1,6 mm. Aus einem auf die seitliche Zuleitung aufgesetzten doppelwandigen mit Öl auf 130°C beheizten Tropftrichter wurde p-Xylylenglykol mit einer Geschwindigkeit von 135 g/h zugetropft.

Temperaturen: Zone I: 400 bis 410°C, Zone II: 360 bis 370°C, Zone III: 355 bis 360°C.

Im oberen Teil des Kühlrohres enstand ein schwarzes teerartiges Produkt, im unteren ein weißlicher Belag. Die Dehydrierung mußte nach kurzer Zeit abgebrochen werden, weil die Katalysatorzone verstopft war.

Der gelblich weiße Belag bestand aus einem Gemisch von Terephthal- und Terephthalaldehydsäure.

### Patentansprüche

1. Verfahren zur Herstellung von Terephthal-, Isophthal- und Phthaldialdehyd, dadurch gekennzeichnet, daß man die entsprechenden Xylylenglykole in Anwesenheit von Sauerstoff oder freien Sauerstoff enthaltenden Gasen und Wasserdampf und in Gegenwart eines Silberkatalysators oder gegebenenfalls Kupferkatalysators in der Dampfphase bei 250 bis 500°C, vorzugsweise 300 bis 450°C, dehydriert.

2. Verfahren nach Anspruch 1, dadurch gekennzeichnet, daß das Molverhältnis von Xylylenglykol zu Sauerstoff 1:0,5 bis 1:1,5, vorzugsweise 1:1 bis 1:1,3, beträgt.

3. Verfahren nach Anspruch 1, dadurch gekennzeichnet, daß das Molverhältnis von Xylylenglykol zu Wasser 1:1 bis 1:100, vorzugsweise 1:5 bis 1:50, beträgt.

4. Verfahren nach einem der Ansprüche 1 oder 2, dadurch gekennzeichnet, daß als sauerstoffenthaltendes Gas Luft verwandt wird.

5. Verfahren nach einem der Ansprüche 1 bis 4, dadurch gekennzeichnet, daß der Dehydrierungskatalysator aus Silber besteht.

6. Verfahren nach Anspruch 5, dadurch gekennzeichnet, daß das Silber auf einen Träger aufgebracht ist.

7. Verfahren nach Anspruch 6, dadurch gekennzeichnet, daß der Träger aus einem inerten Material, bevorzugt aus Bimsstein, besteht.

8. Verfahren nach einem der Ansprüche 1 bis 7, dadurch gekennzeichnet, daß die Dehydrierung bei Normaldruck oder einem geringen Unterdruck durchgeführt wird.

## Claims

1. Process for the production of terephthal-, isophthal-, and phthaldialdehyde, characterised in that the corresponding xylylene glycols are dehydrated in the vapour phase in the presence of oxygen or gases containing free oxygen, and water vapour, and in the presence of a silver catalyst or if necessary a copper catalyst, at 250 to 500°C, preferably 300 to 450°C.

2. Process according to claim 1, characterised in that the mol ratio of xylylene glycol to oxygen is 1:0.5 to 1:1.5, preferably 1:1 to 1:1.3.

3. Process according to claim 1, characterised in that the mol ratio of xylylene glycol to water is 1:1 to 1:100, preferably 1:5 to 1:50.

4. Process according to one of claims 1 or 2, characterised in that air is used as the oxygen-containing gas.

5. Process according to one of claims 1 to 4, characterised in that the dehydration catalyst consists of silver.

6. Process according to claim 5, characterised in that the silver is applied to a carrier.

7. Process according to claim 6, characterised in that the carrier consists of an inert material, preferably pumice stone.

8. Process according to one of claims 1 to 7, characterised in that the dehydration is carried out at normal pressure or at a slightly reduced pressure.

## Revendications

1. Procédé de fabrication de dialdéhydes téréphtalique, isophtalique et phtalique, caractérisé en ce que l'on déshydrogène les xylylèneglycols correspondants en phase vapeur entre 250 et 500°C, de préférence entre 300 et 450°C, en présence d'oxygène ou de gaz contenant de l'oxygène libre et de vapeur d'eau et en présence d'un catalyseur à l'argent ou éventuellement d'un catalyseur au cuivre.

2. Procédé selon la revendication 1, caractérisé en ce que le rapport molaire du xylylèneglycol à l'oxygène est compris entre 1:0,5 et 1:1,5, de préférence entre 1:1 et 1:1,3.

3. Procédé selon la revendication 1, caractérisé en ce que le rapport molaire du xylylèneglycol à l'eau est compris entre 1:1 et 1:100, de préférence entre 1:5 et 1:50.

4. Procédé selon l'une des revendications 1 ou 2, caractérisé en ce que, comme gaz contenant de l'oxygène, on utilise de l'air.

5. Procédé selon l'une quelconque des revendications 1 à 4, caractérisé en ce que le catalyseur de déshydrogénation est formé d'argent.

6. Procédé selon la revendication 5, caractérisé en ce que l'argent est appliqué sur un support.

7. Procédé selon la revendication 6, caractérisé en ce que le support est formé d'une matière inerte, de préférence de pierre ponce.

8. Procédé selon l'une quelconque des revendications 1 à 7, caractérisé en ce que l'on conduit la déshydrogénation à la pression normale ou sous une légère dépression.